# EUROPEAN PATENT SPECIFICATION

(11) **EP 0 301 638 B1**
(45) Date of publication and mention of the grant of the patent: **08.12.1993**
(21) Application number: 88201524.1
(22) Date of filing: 15.07.1988
(51) Int. Cl.: C07C 271/06, C07C 271/40, C07D 307/83, C07D 317/50, C07D 317/20

(54) **Improved process for producing n-alkyl-carbamates**
Verfahren zur Herstellung von N-Alkylcarbamaten
Procédé de préparation de N-alkylcarbamates

(30) Priority: 31.07.1987 IT 2156187
(43) Date of publication of application: 01.02.1989
(73) Proprietor: ENICHEM SYNTHESIS S.p.A., 90139 Palermo (IT)
(72) Inventor: Mizia, Franco, I-20139 Milan (IT); Rivetti, Franco, I-36015 Schio Vicenza (IT); Romano, Ugo, I-20059 Vimercate Milan (IT)
(74) Representative: Fusina, Gerolamo

(56) References cited:
- DD-A- 250 114
- DE-A- 1 720 633
- GB-A- 2 155 487

## Description

The present invention relates to an improved process for producing N-alkyl-carbamates by means of the reaction of a phenol or of a naphthol with an alkyl isocyanate.

N-alkyl-carbamates are valuable products and many of them display an useful activity as plant disease control agents, such as, e.g., 2,3-dihydro-2,2-dimethyl-benzofuran-7-yl N-methyl-carbamate (known as CARBOFURAN), 1-naphthyl N-methyl-carbamate (known as CARBARYL) and 2-isopropoxy-phenyl N-methyl-carbamate (known as PROPOXUR).

N-alkyl-carbamates are obtained, according to the prior art, by reacting an alkyl isocyanate, in particular methyl isocyanate, with a phenol or a naphthol, by operating in an inert organic solvent and in the presence of a basic catalyst. For this purpose, reference is made to R.J. Kuhr and H.D.Dorough, "Carbamates Insecticides; Chemistry, Biochemistry and Toxicology", CRC Press (1977) and to the disclosure of US-A 4 390 710 and of EP-A. 200 249.

The catalysts used in said reaction have a basic nature, and are normally selected from tertiary amines (e.g., triethylamine and triisopropylamine); heterocyclic bases (e.g., pyridine, 4 -dimethyl-amino-pyridine and N-methyl-imidazole); alkali-metal alkoxides (e.g., sodium methoxide and sodium ethoxide); alkali-metal carbonates (e.g., sodium carbonate); and derivatives of such metals as tin and titanium (e.g., dibutyl-tin dilaurate and dibutyl-tin diacetate).

The preferred catalysts are the tertiary amines and tin derivatives.

In practice, the reaction between a phenol or a naphthol and an alkyl-isocyanate is carried out in the presence of said catalysts, by operating at relatively low temperatures, in an organic solvent, and using a mutual stoichiometric ratio of the reactants, or a slight excess of the phenol or naphthol over the stoichiometric amount. Under these conditions, the reaction proceeds regularly, with high yields to the useful reaction product. The drawback which affects said reaction consists in the long times required for completing, or substantially completing, the same reaction, in particular in order to reduce to negligibly low values (less than 100 ppm - parts per million by weight) the residual alkyl isocyanate content in the reaction medium.

Such a matter of fact constitutes a disadvantage from both standpoints of process cheapness, and of the extremely high toxicity of alkyl isocyanates in general, and of methyl isocyanate in particular.

The present Applicant has found now that the drawbacks affecting the prior art can be overcome by means of the adoption of particular catalysts which enable unexpectedly high reaction speeds between the phenol or the naphthol and the alkyl-isocyanate to be reached. Therefore, according to the present invention, it results possible the concentration of the alkyisocyanate to be reduced to negligibly low values, by operating with short reaction times, in systems using stoichiometric, or approximately stoichiometric, amounts of the reactants.

In accordance therewith, the present invention relates to a process for preparing N-alkyl-carbamates by catalytically reacting an alkyl-isocyanate with a phenol, or a naphthol, in an inert organic solvent characterized in that said reaction is carried out at a molar ratio of the phenol or the naphthol to the alkylisocyanate of from 1:1 to 1,1:1, at a temperature of from 0°C to 50°C, and in the presence of an amount of from 0,0001 mol to 0,05 mol per mol of the phenol or the naphthol concerned, of a catalyst selected from the bicyclic amidines, having the general formula: 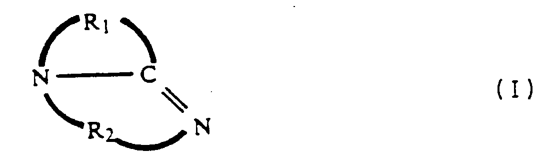
or from the bicyclic guanidines containing a stretched tertiary nitrogen bonded to a ketoimine carbon atom, having the general formula: 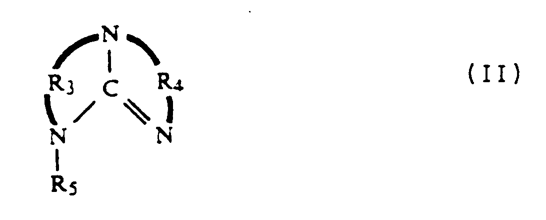
wherein:
R₁ and R₂ are, independently, C₃-C₅ unsubstituted alkylenes or substituted alkylenes, the substituents whereof are one or more C₁-C₅ alkyls or phenyls;
R₃ and R₄ are, independently, C₃ or C₄ unsubstituted alkylenes or substituted alkylenes, the substituents whereof are one or more C₁-C₅ alkyls or phenyls, and
R₅ is selected from the C₁-C₅ alkyls or the alkylaryls.

Specific examples of catalysts are:
1,5-diazabicyclo (4.3.0) non-5-ene 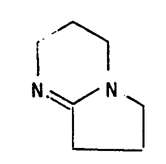
3-phenyl-1,5-diazabicyclo (4.3.0) non-5-ene 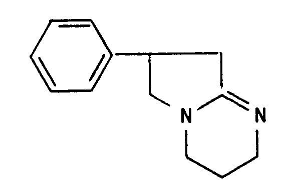
1,8-diazabicyclo (5.4.0) undec-7-ene 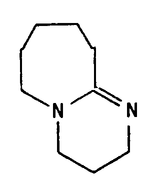
7-methyl-1,5,7-triazabicyclo (4.4.0) dec-5-ene 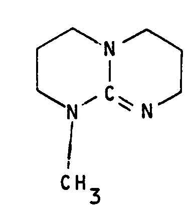
1,8-Diazabicyclo (5.4.0) undec-7-ene can be prepared by making caprolactam and acrylonitrile react with each other (Michael reaction) and catalytically reducing the so obtained reaction product, as described by H. Oediger and H. Moller in Angew. Chem. 79, 53 (1967).

1,5-Diazabicyclo (4.3.0) non-5-ene can be prepared in a similar way by starting from butyrolactam.

Other amidines useful for the intended purpose are described in Tetrahedron Letters, 51, 5175-7 (1967).

The reaction between the phenol or naphthol and the alkyl-isocyanate can be represented by the following equation: 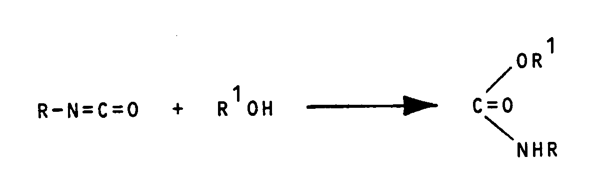
The alkyl-isocyanates (R-N=C=O) useful for the intended purpose are those wherein R represents an alkyl group containing from 1 to 4 carbon atoms, and preferably a methyl group.

Examples of R¹OH compounds, useful for the intended purpose, are phenol; substituted phenol, with from 1 to 3 substituents, which may be either equal to, or different from, one another, selected from alkyl, oxy-alkyl, thio-alkyl, amino-alkyl, alkylene-oxy-alkyl, alkylene-thio-alkyl, alkylene-amino-alkyl groups, wherein the alkyl group, which may be either linear or branched, contains from 1 to 5 carbon atoms (preferably from 1 to 3 carbon atoms) and the alkylene group contains 1 or 2 carbon atoms (it is preferably methylene); 1-naphthol; 2-naphthol; 2,3-dihydro-2,2-dimethyl-benzofuran-7-ol; 2,2-dimethyl-1,3-benzodioxol-4-ol; and 2-(1,3-dioxolan-2-yl)-phenol.

Examples of preferred R¹OH compounds for the purposes of the present invention are: 3,5-xylenol; 3,4-xylenol; 2-isopropyl-phenol; 2-isopropoxy-phenol; 2-(ethylthiometyl)-phenol; 2-cresol; 3-isopropyl-5-methyl-phenol; 4-methyl-thio-3,5-dimethyl-phenol; 4-dimethylamino-3-methyl-phenol; 1-naphthol; 2,3-dihydro-2,2-dimethylbenzofuran-7-ol; and 2,2-dimethyl-1,3-benzodioxol-4-ol.

According to the process of the present invention, the reaction is carried out in solution in an inert organic solvent and the solvents suitable for the intended purpose can be selected from aromatic hydrocarbons, such as benzene, toluene and xylene; ketones, such as acetone, methyl-ethyl-ketone and methyl-isobutyl-ketone; esters, such as ethyl acetate, dimethyl carbonate and diethyl carbonate; chlorinated aliphatic hydrocarbons, such as chloroform, methylene chloride, carbon tetrachloride and dichloroethane; and ethers, such as diethyl ether and tetrahydrofuran.

The reaction temperatures are within the range of from 0° to 50°C.

Furthermore, the reaction is carried out with an equimolar or nearly equimolar ratio of the phenol or naphthol reactant to the alkyl-isocyanate reactant, i.e. with a molar ratio within the range of from 1/1 to 1.1/1.

The amount of catalyst used for the reaction is within the range of from 0.0001 to 0.05 mol per each mol of the phenol or naphthol compound. The preferred catalyst amounts are comprised within the range of from 0.0005 to 0.02 mol per each mol of the phenol or naphthol compound.

The way how the reactants are placed into contact is not critic, however, in the preferred form of practical embodiment the reaction is carried out by dissolving the phenol or naphthol, besides the catalyst, in the selected organic solvent, and to the so obtained solution the alkyl isocyanate is gradually added until a molar ratio of the phenol or naphthol to said alkyl-isocyanate is reached, which is comprised within the above stated range of values.

A technique of such a kind is disclosed, e.g., in US-A. 4 659 845.

By means of the catalysts of the present invention, rates of reaction between the phenol or naphthol and the alkyl-isocyanate are reached, which are generally from 100 to 1,000 times higher than those which can be obtained by means of the catalysts known in the prior art, with the molar catalyst concentration, and the other reaction conditions being the same.

In order to better evidence the activity of the catalysts according to the present invention, the values can be compared of the constants of rate of carbamate formation (Kabs) from 2,3-dihydro-2,2-dimethyl-benzofuran-7-ol with methyl isocyanate, which are obtained by operating at 25°C in toluene as the solvent, by using various concentrations of:
- 1,8-diazabicyclo (5,4,0) undec-7-eme (DBU),
- 1,5-diazabicyclo (5,4,0) non-5-ene (DBN) and
- 7-methyl-1,5,7-triazabicyclo (4,4,0) dec-5-ene (MTBD) and of the catalyst known from the prior art triethylamine (TEA).

| DBU | Kabs |
|---|---|
| 2 . 10⁻³ mol/litre | 5,63 litres.mol⁻¹.min⁻¹ |
| 8 . 10⁻³ mol/litre | 22.2 litres.mol⁻¹.min⁻¹ |
| 2 . 10⁻² mol/litre | 27.7 litres.mol⁻¹.min⁻¹ |

| DBN | Kabs |
|---|---|
| 1 . 10⁻³ mol/litre | 4.5 litres.mol⁻¹.min⁻¹ |
| 8 . 10⁻³ mol/litre | 15.5 litres.mol⁻¹.min⁻¹ |

| MIBD | Kabs |
|---|---|
| 3.4 . 10⁻⁴ mol/litre | 12.5 litres.mol⁻¹.min⁻¹ |
| 2.3 . 10⁻³ mol/litre | 31.0 litres.mol⁻¹.min⁻¹ |

| TEA | Kabs |
|---|---|
| 2.6 . 10⁻³ mol/litre | 6.89.10⁻³ litres.mol⁻¹.min⁻¹ |
| 8.15. 10⁻³ mol/litre | 2.9 .10⁻² litres.mol⁻¹.min⁻¹ |
| 1.3 . 10⁻² mol/litre | 5.0 .10⁻² litres.mol⁻¹.min⁻¹ |

The following experimental examples are illustrative and not limitative of the present invention.

### Example 1

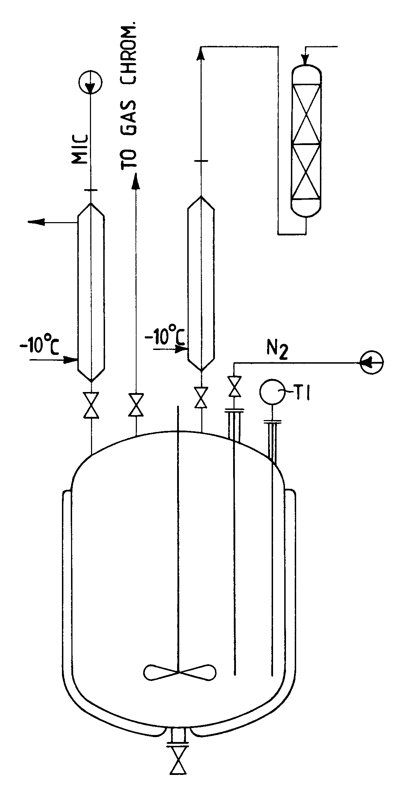

The equipment reported in the figure of the hereto attached drawing table is used, which comprises a reactor, equipped with a stirrer, which operates at atmospheric pressure and is connected through a condenser to a demister. The operating temperature is 20°C.

Methyl isocyanate is fed as a liquid (-10°C), at a flow rate of 6.93 g/hour, to the reactor, to which: 2,3-dihydro-2,2-dimethyl-benzofuran-7-ol (63.2 g), toluene (250 g) and 1,8-diazabicyclo (5.4.0) undec-7-ene (0.045 g) were previously charged.

The methyl isocyanate stream is fed for 3 hours. After this time period, the flow is discontinued and the reactor is separated from the feed line.

The residual amount of methyl isocyanate in the solution is determined at regular time intervals, by means of the chromatography of a constant nitrogen stream saturated, at room temperature, with methyl isocyanate and toluene (the reactor's vapour phase).

By means of a standard curve, the concentration of methyl isocyanate in the liquid phase is obtained.

The values of residual methyl isocyanate concentrations, expressed as parts per million parts by weight (MIC ppm) starting from the time of interruption of reactor feed are:

| Time (minutes) | MIC ppm |
|---|---|
| 0 | 1,600 |
| 10^{I} | 160 |
| 15^{I} | 57 |
| 20^{I} | 21 |

By operating as previously disclosed, but using triethylamine as the catalyst, fed in an amount of 0.45 g (i.e., one order of magniture larger than of 1,8-diazabicyclo (5.4.0) undec-7-ene), the following results are obtained:

| Time (minutes) | MIC ppm | mol/litre |
|---|---|---|
| 0 | 25,500 | 0.402 |
| 10^{I} | 22,000 | 0.347 |
| 15^{I} | 20,100 | 0.318 |
| 20^{I} | 18,800 | 0.296 |

### EXAMPLE 2

The process is carried out as described in Example 1, by charging to the reactor:
- 2,3-dihydro-2,2-dimethyl-benzofuran-7-ol: 63.2 g
- toluene : 250 g
- 1,5-diazobicyclo (4.3.0) non-5-ene : 0.036 g
The values of residual methyl isocyanate concentration are:

| Time (minutes) | MIC ppm | mol/litre |
|---|---|---|
| 0 | 1,120 | 0.017 |
| 5^{I} | 150 | 2.37.10⁻³ |
| 10^{I} | 30 | 4.37.10⁻⁴ |

### EXAMPLE 3

The process is carried out as described in Example 1, by charging to the reactor:

| | |
|---|---|
| - 1-naphthol | 55.5 g |
| - toluene | 500 g |
| - 1,8-diazabicyclo (5.4.0) undec-7-ene | 0.174 g |

The values of residual methyl isocyanate concentration are:

| Time (minutes) | MIC ppm | mol/litre |
|---|---|---|
| 0 | 1,380 | 0.022 |
| 5^{I} | 352 | 5.5.10⁻³ |
| 10^{I} | 120 | 1.9.10⁻³ |
| 15^{I} | 40 | 6.10⁻⁴ |

## Claims

1. Process for preparing N-alkyl-carbamates by catalytically reacting an alkyl-isocyanate with a phenol, or a naphthol, in an inert organic solvent characterized in that said reaction is carried out at a molar ratio of the phenol or the naphthol to the alkylisocyanate of from 1:1 to 1,1:1, at a temperature of from 0°C to 50°C, and in the presence of an amount of from 0,0001 mol to 0,05 mol per mol of the phenol or the naphthol concerned, of a catalyst selected from the bicyclic amidines, having the general formula: 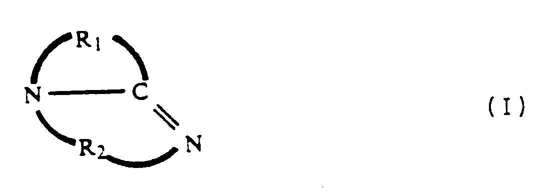 or from the bicyclic guanidines containing a stretched tertiary nitrogen bonded to a ketoimine carbon atom, having the general formula: 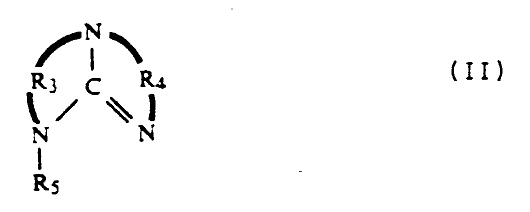 wherein:
R₁ and R₂ are, independently, C₃-C₅ unsubstituted alkylenes or substituted alkylenes, the substituents whereof are one or more C₁-C₅ alkyls or phenyls;
R₃ and R₄ are, independently, C₃ or C₄ unsubstituted alkylenes or substituted alkylenes, the substituents whereof are one or more C₁-C₅ alkyls or phenyls, and
R₅ is selected from the C₁-C₅ alkyls or the alkylaryls.

2. Process according to Claim 1, wherein the catalyst is selected from:
1,5-diazabicyclo-(4.3.0)-non-5-ene;
3-phenyl-1,5-diazabicyclo-(4.3.0)-non-5-ene;
1,8-diazabicyclo-(5.4.0)-undec-7-ene, and
7-methyl-1,5,7-triazabicyclo-(4.4.0)-dec-5-ene.

3. Process according to Claim 1, wherein the amount of the catalyst is from 0,0005 mol to 0,02 mol of the catalyst concerned per mol of the phenol or the naphthol concerned.

4. Process according to Claim 1, wherein said phenol or naphthol is selected from:
phenol, substituted phenols carrying from 1 to 3 substituents, equal to, or different from each other and selected from:
alkyls,
oxyalkyls,
thioalkyls,
aminoalkyls,
alkylenoxyalkyls,
alkylenethioalkyls, and
alkyleneaminoalkyls,
the alkyl of the latter being a linear or a branched C₁-C₅ alkyl, the alkylene being a C₁-C₂ alkylene;
1-naphthol;
2-naphthol;
2,3-dihydro-2,2-dimethyl-benzofuran-7-ol;
2,2-dimethyl-1,3-benzodioxol-4-ol, and
2-(1,3-dioxolan-2-yl)phenol.

5. Process according to Claim 4, wherein said phenol or naphthol is selected from
3,4-xylenol,
3,5-xylenol,
2-isopropyl-phenol,
2-isopropoxy-phenol,
2-(ethylthiomethyl)-phenol,
2-cresol,
3-isopropyl-5-methyl-phenol,
4-methyl-thio-3,5-dimethyl-phenol, and
4-dimethylamino-3-methyl-phenol.

6. Process according to Claim 1, wherein the alkyl of said alkyl isocyanate is a C₁-C₄ alkyl.

7. Process according to Claims 1 and 6, wherein the alkylisocyanate is methyl isocyanate.

8. Process according to Claim 1, wherein said inert organic solvent is selected from the aromatic hydrocarbons, the ketones, the esters, the chlorinated hydrocarbons and the ethers.

## Patentansprüche

1. Verfahren zur Herstellung von N-Alkylcarbamaten durch katalytische Umsetzung eines Alkylisocyanats mit einem Phenol oder einem Naphthol in einem inerten organischen Lösungsmittel, dadurch gekennzeichnet, daß diese Umsetzung bei einem Molverhältnis des Phenols oder Naphthols zu dem Alkylisocyanat von 1:1 bis 1,1:1 bei einer Temperatur von 0°C bis 50°C und in Gegenwart einer Menge von 0,0001 Mol bis 0,05 Mol je Mol des entsprechenden Phenols oder Naphthols eines Katalysators, ausgewählt unter den bicyklischen Amidinen mit der allgemeinen Formel: 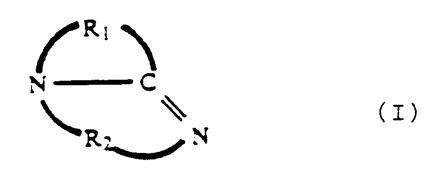 oder unter den bicyklischen Guanidinen mit einem Gehalt an einem an ein Ketoimin-Kohlenstoffatom gebundenen gespannten tertiären Stickstoffatom mit der allgemeinen Formel: 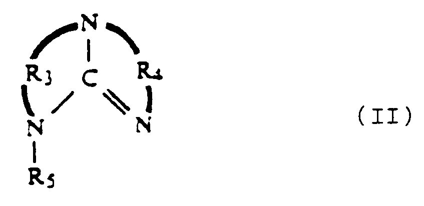 ausgeführt wird, worin:
R₁ und R₂ unabhängig voneinander unsubstituierte oder substituierte C₃-C₅Alkylene darstellen, deren Substituenten ein oder mehrere C₁-C₅Alkyle oder Phenyle sind;
R₃ und R₄ unabhängig voneinander unsubstituierte oder substituierte C₃- oder C₄-Alkylene sind, deren Substituenten ein oder mehrere C₁-C₅Alkyle oder Phenyle sind, und
R₅ unter C₁-C₅Alkylen oder Alkylarylen ausgewählt ist.

2. Verfahren nach Anspruch 1, worin der Katalysator unter 1,5-Diazabicyclo-(4.3.0.)-non-5-en, 3-Phenyl-1,5-diazabicyclo-(4.3.0)-non-5-en, 1,8-Diazabicyclo-(5.4.0)-undec-7-en und 7-Methyl-1,5,7-triazabicyclo-(4,4,0)-dec-5-en ausgewählt ist.

3. Verfahren nach Anspruch 1, worin die Katalysatormenge von 0,0005 Mol bis 0,02 Mol des jeweiligen Katalysators je Mol eingesetztes Phenol oder Naphthol beträgt.

4. Verfahren nach Anspruch 1, worin das Phenol oder Naphthol unter Phenol, substituierten Phenolen mit 1 bis 3 Substituenten, die gleich oder voneinander verschieden sind und unter Alkyl, Oxyalkyl, Thioalkyl, Aminoalkyl, Alkylenoxyalkyl, Alkylenthioalkyl und Alkylenaminoalkyl ausgewählt sind, wobei der Alkylteil der Letztgenannten ein lineares oder verzweigtes C₁-C₅Alkyl ist und Alkylen ein C₁-C₂Alkylen ist, 1-Naphthol, 2-Naphthol, 2,3-Dihydro-2,2-dimethyl-benzofuran-7-ol, 2,2-Dimethyl-1,3-benzodioxol-4-ol und 2-(1,3-dioxolan-2-yl)phenol ausgewählt ist.

5. Verfahren nach Anspruch 4, worin das Phenol oder Naphthol unter 3,4-Xylenol, 3,5-Xylenol, 2-Isopropylphenol, 2-Isopropoxyphenol, 2-(Ethylthiomethyl)phenol, 2-Cresol, 3-Isopropyl-5-methylphenol, 4-Methylthio-3,5-dimethylphenol und 4-Dimethylamino-3-methylphenol ausgewählt ist.

6. Verfahren nach Anspruch 1, worin das Alkyl des Alkylisocyanats ein C₁-C₄Alkyl ist.

7. Verfahren nach den Ansprüchen 1 und 6, worin das Alkylisocyanat Methylisocyanat ist.

8. Verfahren nach Anspruch 1, worin das inerte organische Lösungsmittel unter aromatischen Kohlenwasserstoffen, Ketonen, Estern, chlorierten Kohlenwasserstoffen und Ethern ausgewählt ist.

## Revendications

1. Procédé de préparation de N-alkyl-carbamates par réaction catalytique d'un isocyanate d'alkyle avec un phénol ou un naphtol, dans un solvant organique inerte, caractérisé en ce que ladite réaction est effectuée avec un rapport molaire du phénol ou du naphtol à l'isocyanate d'alkyle valant de 1:1 à 1,1:1, à une température de 0°C à 50°C, et en présence d'une quantité représentant de 0,0001 mole à 0,05 mole, par mole du phénol ou du naphtol concerné, d'un catalyseur choisi parmi les amidines bicycliques de formule générale : 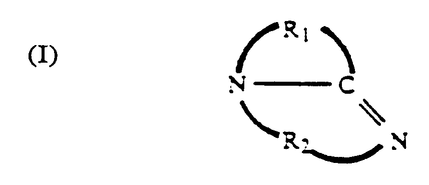 et les guanidines bicycliques contenant un atome d'azote tertiaire contraint et lié à un atome de carbone cétoiminique, de formule générale : 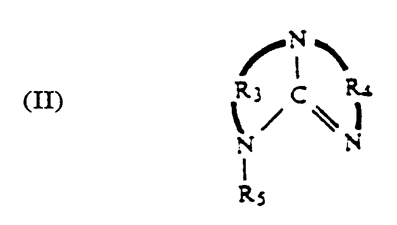 formules dans lesquelles :
R₁ et R₂ représentent, indépendamment, des groupes alkylène en C₃-C₅ non substitués ou des groupes alkylène substitués dont les substituants sont un ou plusieurs groupes alkyle en C₁-C₅ ou phényle,
R₃ et R₄ représentent, indépendamment, des groupes alkylène en C₃ ou C₄ non substitués ou des groupes alkylène substitués dont les substituants sont un ou plusieurs groupes alkyle en C₁-C₅ ou phényle, et
R₅ est choisi parmi les groupes alkyle en C₁-C₅ et les groupes alkyl-aryle.

2. Procédé conforme à la revendication 1, dans lequel le catalyseur est choisi parmi :
le 1,5-diazabicyclo-(4.3.0)-non-5-ène,
le 3-phényl-1,5-diazabicyclo-(4.3.0)-non-5-ène,
le 1,8-diazabicyclo-(5.4.0)-undéc-7-ène, et
le 7-méthyl-1,5,7-triazabicyclo-(4.4.0)-déc-5-ène.

3. Procédé conforme à la revendication 1, dans lequel la quantité de catalyseur vaut de 0,0005 mole à 0,02 mole du catalyseur concerné par mole du phénol ou naphtol concerné.

4. Procédé conforme à la revendication 1, dans lequel ledit phénol ou naphtol est choisi parmi :
le phénol,
les phénols substitués portant de 1 à 3 substituants, identiques ou différents les uns des autres et choisis parmi les groupes alkyle, oxyalkyle, thioalkyle, aminoalkyle, alkylène-oxyalkyle, alkylène-thioalkyle et alkylène-aminoalkyle, le groupe alkyle de ce dernier étant un groupe alkyle linéaire ou ramifié en C₁-C₅ et le groupe alkylène étant un groupe alkylène en C₁-C₂,
le 1-naphtol,
le 2-naphtol,
le 2,3-dihydro-2,2-diméthyl-benzofurane-7-ol,
le 2,2-diméthyl-1,3-benzodioxole-4-ol, et
le 2-(1,3-dioxolane-2-yl)phénol.

5. Procédé conforme à la revendication 4, dans lequel ledit phénol ou naphtol est choisi parmi :
le 3,4-xylénol,
le 3,5-xylénol,
le 2-isopropyl-phénol,
le 2-isopropoxy-phénol,
le 2-(éthylthiométhyl)-phénol,
le 2-crésol,
le 3-isopropyl-5-méthyl-phénol,
le 4-méthylthio-3,5-diméthyl-phénol, et
le 4-diméthylamino-3-méthyl-phénol.

6. Procédé conforme à la revendication 1, dans lequel le groupe alkyle dudit isocyanate d'alkyle est un groupe alkyle en C₁-C₄.

7. Procédé conforme aux revendications 1 et 6, dans lequel l'isocyanate d'alkyle est de l'isocyanate de méthyle.

8. Procédé conforme à la revendication 1, dans lequel ledit solvant inerte est choisi parmi les hydrocarbures aromatiques, les cétones, les esters, les hydrocarbures chlorés et les éthers.
